# EUROPÄISCHE PATENTANMELDUNG

(11) **EP 1 975 241 A1**
(43) Veröffentlichungstag der Anmeldung: **01.10.2008**
(21) Anmeldenummer: 08102174.3
(22) Anmeldetag: 29.02.2008
(51) Int. Cl.: C12P 13/08, C12P 13/22

(54) **Verfahren zur Herstellung von L-Aminosäuren unter Verwendung von verbesserten Stämmen der Familie Enterobacteriaceae**

(30) Priorität: 29.03.2007 DE 102007015582
(71) Anmelder: Evonik Degussa GmbH, 45128 Essen (DE)
(72) Erfinder: Rieping, Mechthild, Dr., 33619, Bielefeld (DE); Schneider, Frank, Dr., 33790, Halle (DE); Skroblin, Cornelia, 32139, Spenge (DE)

(57) **Zusammenfassung**

Die Erfindung betrifft ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man das malI-Gen, für das Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

SEQUENZPROTOKOLL

<110>  Evonik Degussa GmbH

<120>  Verfahren zur Herstellung von L-Aminosäuren unter Verwendung von
       verbesserten Stämmen der Familie Enterobacteriaceae

<130>  200700192

<160>  16

<170>  PatentIn version 3.3

<210>  1
<211>  1029
<212>  DNA
<213>  Escherichia coli


<220>
<221>  CDS
<222>  (1)..(1029)
<223>  malI-Kodierregion

<400>  1
atg gct acc gcc aaa aaa ata acc att cat gat gtt gcg ctg gct gcg       48
Met Ala Thr Ala Lys Lys Ile Thr Ile His Asp Val Ala Leu Ala Ala
1               5                   10                  15

ggc gtg tcg gta agt acc gtt tcg ctg gtg ctt agt ggc aaa ggg cga       96
Gly Val Ser Val Ser Thr Val Ser Leu Val Leu Ser Gly Lys Gly Arg
            20                  25                  30

atc tct acc gcc aca gga gaa cgc gtt aac gcc gcc att gaa gag ctg      144
Ile Ser Thr Ala Thr Gly Glu Arg Val Asn Ala Ala Ile Glu Glu Leu
        35                  40                  45

gga ttt gtg cgc aat cgc cag gcg tcg gcg ctg cgc ggc ggg caa agc      192
Gly Phe Val Arg Asn Arg Gln Ala Ser Ala Leu Arg Gly Gly Gln Ser
    50                  55                  60

ggc gtc att ggt ttg atc gtc cgt gat tta tct gcg ccg ttt tac gcc      240
Gly Val Ile Gly Leu Ile Val Arg Asp Leu Ser Ala Pro Phe Tyr Ala
65                  70                  75                  80

gaa ttg acg gcc gga ttg acg gaa gct ctg gaa gcg cag gga cgg atg      288
Glu Leu Thr Ala Gly Leu Thr Glu Ala Leu Glu Ala Gln Gly Arg Met
                85                  90                  95

gtt ttt ttg ctt cac ggc ggt aaa gac ggt gag cag ctg gca cag cgg      336
Val Phe Leu Leu His Gly Gly Lys Asp Gly Glu Gln Leu Ala Gln Arg
            100                 105                 110

ttt tca ctg tta ctg aat cag ggt gtc gat ggt gtg gta att gcc gga      384
Phe Ser Leu Leu Leu Asn Gln Gly Val Asp Gly Val Val Ile Ala Gly
        115                 120                 125

gct gca gga agt agc gat gac ctg cga cgg atg gca gaa gaa aaa gct      432
Ala Ala Gly Ser Ser Asp Asp Leu Arg Arg Met Ala Glu Glu Lys Ala
    130                 135                 140

atc ccg gtg att ttc gct tcc cgt gcc agt tat ctt gat gat gtt gat      480
Ile Pro Val Ile Phe Ala Ser Arg Ala Ser Tyr Leu Asp Asp Val Asp
145                 150                 155                 160

acg gtt cgc ccg gac aac atg cag gct gca cag ttg ttg acg gag cat      528

Thr Val Arg Pro Asp Asn Met Gln Ala Ala Gln Leu Leu Thr Glu His
                165                 170                 175

ctc att cgc aat ggg cat cag cgg atc gcc tgg ctg gga ggg caa agt      576
Leu Ile Arg Asn Gly His Gln Arg Ile Ala Trp Leu Gly Gly Gln Ser
            180                 185                 190

tcc tca tta acc cgt gca gaa cgg gtt ggg ggc tat tgt gca act cta      624
Ser Ser Leu Thr Arg Ala Glu Arg Val Gly Gly Tyr Cys Ala Thr Leu
        195                 200                 205

cta aaa ttt ggc ctg ccg ttt cat agc gat tgg gtg ttg gag tgc act      672
Leu Lys Phe Gly Leu Pro Phe His Ser Asp Trp Val Leu Glu Cys Thr
    210                 215                 220

tcc agc cag aag caa gcc gcg gaa gct atc acg gcg ctt tta cgt cat      720
Ser Ser Gln Lys Gln Ala Ala Glu Ala Ile Thr Ala Leu Leu Arg His
225                 230                 235                 240

aac ccg acc atc agt gcc gtg gtt tgc tat aac gaa act att gcg atg      768
Asn Pro Thr Ile Ser Ala Val Val Cys Tyr Asn Glu Thr Ile Ala Met
                245                 250                 255

ggg gca tgg ttt ggt ttg ctc aaa gca ggg cgg caa agc ggg gaa agc      816
Gly Ala Trp Phe Gly Leu Leu Lys Ala Gly Arg Gln Ser Gly Glu Ser
            260                 265                 270

gga gtc gat cgt tac ttt gag caa cag gtt tcg ctg gcg gca ttt acc      864
Gly Val Asp Arg Tyr Phe Glu Gln Gln Val Ser Leu Ala Ala Phe Thr
        275                 280                 285

gat gcg aca cca acc aca ctt gat gat ata cct gtt acc tgg gcc agc      912
Asp Ala Thr Pro Thr Thr Leu Asp Asp Ile Pro Val Thr Trp Ala Ser
    290                 295                 300

acg cca gcg cgg gaa ctt ggt atc aca ctt gcg gat cgc atg atg caa      960
Thr Pro Ala Arg Glu Leu Gly Ile Thr Leu Ala Asp Arg Met Met Gln
305                 310                 315                 320

aaa atc acc cat gaa gag acg cat tca cgc aat ctt att att ccc gcc     1008
Lys Ile Thr His Glu Glu Thr His Ser Arg Asn Leu Ile Ile Pro Ala
                325                 330                 335

cgg ctc att gca gcg aaa taa                                         1029
Arg Leu Ile Ala Ala Lys
            340


<210>  2
<211>  342
<212>  PRT
<213>  Escherichia coli

<400>  2

Met Ala Thr Ala Lys Lys Ile Thr Ile His Asp Val Ala Leu Ala Ala
1               5                   10                  15


Gly Val Ser Val Ser Thr Val Ser Leu Val Leu Ser Gly Lys Gly Arg
            20                  25                  30


Ile Ser Thr Ala Thr Gly Glu Arg Val Asn Ala Ala Ile Glu Glu Leu
        35                  40                  45

Gly Phe Val Arg Asn Arg Gln Ala Ser Ala Leu Arg Gly Gly Gln Ser
    50                  55                  60

Gly Val Ile Gly Leu Ile Val Arg Asp Leu Ser Ala Pro Phe Tyr Ala
65                  70                  75                  80

Glu Leu Thr Ala Gly Leu Thr Glu Ala Leu Glu Ala Gln Gly Arg Met
                85                  90                  95

Val Phe Leu Leu His Gly Gly Lys Asp Gly Glu Gln Leu Ala Gln Arg
            100                 105                 110

Phe Ser Leu Leu Leu Asn Gln Gly Val Asp Gly Val Val Ile Ala Gly
        115                 120                 125

Ala Ala Gly Ser Ser Asp Asp Leu Arg Arg Met Ala Glu Glu Lys Ala
    130                 135                 140

Ile Pro Val Ile Phe Ala Ser Arg Ala Ser Tyr Leu Asp Asp Val Asp
145                 150                 155                 160

Thr Val Arg Pro Asp Asn Met Gln Ala Ala Gln Leu Leu Thr Glu His
                165                 170                 175

Leu Ile Arg Asn Gly His Gln Arg Ile Ala Trp Leu Gly Gly Gln Ser
            180                 185                 190

Ser Ser Leu Thr Arg Ala Glu Arg Val Gly Gly Tyr Cys Ala Thr Leu
        195                 200                 205

Leu Lys Phe Gly Leu Pro Phe His Ser Asp Trp Val Leu Glu Cys Thr
    210                 215                 220

Ser Ser Gln Lys Gln Ala Ala Glu Ala Ile Thr Ala Leu Leu Arg His
225                 230                 235                 240

Asn Pro Thr Ile Ser Ala Val Val Cys Tyr Asn Glu Thr Ile Ala Met
                245                 250                 255

Gly Ala Trp Phe Gly Leu Leu Lys Ala Gly Arg Gln Ser Gly Glu Ser
            260                 265                 270

Gly Val Asp Arg Tyr Phe Glu Gln Gln Val Ser Leu Ala Ala Phe Thr
        275                 280                 285

Asp Ala Thr Pro Thr Thr Leu Asp Asp Ile Pro Val Thr Trp Ala Ser
    290                 295                 300

Thr Pro Ala Arg Glu Leu Gly Ile Thr Leu Ala Asp Arg Met Met Gln

305                 310                 315                 320

Lys Ile Thr His Glu Glu Thr His Ser Arg Asn Leu Ile Ile Pro Ala
                325                 330                 335

Arg Leu Ile Ala Ala Lys
            340

<210>  3
<211>  1029
<212>  DNA
<213>  Shigella flexneri

<220>
<221>  CDS
<222>  (1)..(1029)
<223>  malI-Kodierregion

<400>  3
atg gct acc gcc aaa aaa ata acc att cat gat gtt gcg ctg gct gcg       48
Met Ala Thr Ala Lys Lys Ile Thr Ile His Asp Val Ala Leu Ala Ala
1               5                   10                  15

ggc gtg tcg gta agt acc gtt tcg ctg gtg ctt aat ggc aaa ggg cga       96
Gly Val Ser Val Ser Thr Val Ser Leu Val Leu Asn Gly Lys Gly Arg
            20                  25                  30

atc tct acc gcc aca gga gaa cgc gtt aac gcc gcc att gaa gag ctg      144
Ile Ser Thr Ala Thr Gly Glu Arg Val Asn Ala Ala Ile Glu Glu Leu
        35                  40                  45

gga ttt gtg cgc aat cgc cag gcg tcg gcg ctg cgc ggc ggg caa agc      192
Gly Phe Val Arg Asn Arg Gln Ala Ser Ala Leu Arg Gly Gly Gln Ser
    50                  55                  60

ggc gtc att ggt ttg atc gtc cgt gat tta tct gcg ccg ttt tac gcc      240
Gly Val Ile Gly Leu Ile Val Arg Asp Leu Ser Ala Pro Phe Tyr Ala
65                  70                  75                  80

gaa ttg acg gcc gga ttg acg gaa gct ctg gaa gca cag gga cgg atg      288
Glu Leu Thr Ala Gly Leu Thr Glu Ala Leu Glu Ala Gln Gly Arg Met
                85                  90                  95

gtt ttt ttg ctt cac ggc ggt aaa gac ggc gag cag ctg gca cag cgg      336
Val Phe Leu Leu His Gly Gly Lys Asp Gly Glu Gln Leu Ala Gln Arg
            100                 105                 110

ttt tca ctg tta ctg aat cag ggt gtc gat ggt gtg gta att gcc ggg      384
Phe Ser Leu Leu Leu Asn Gln Gly Val Asp Gly Val Val Ile Ala Gly
        115                 120                 125

gct gca gga agc agc gat gac ctg cgg cgg atg gca gaa gaa aaa gct      432
Ala Ala Gly Ser Ser Asp Asp Leu Arg Arg Met Ala Glu Glu Lys Ala
    130                 135                 140

atc ccg gtg att ttc gct tcc cgt gcc agt tat ctt gat gat gtt gat      480
Ile Pro Val Ile Phe Ala Ser Arg Ala Ser Tyr Leu Asp Asp Val Asp
145                 150                 155                 160

acg gtt cgc ccg gac aac atg cag gct gca cag ttg ttg acg gag cat      528
Thr Val Arg Pro Asp Asn Met Gln Ala Ala Gln Leu Leu Thr Glu His
                165                 170                 175

ctc att cgc aat ggg cat cag cgg atc gcc tgg ctg gga ggg caa agt 576
Leu Ile Arg Asn Gly His Gln Arg Ile Ala Trp Leu Gly Gly Gln Ser
            180                 185                 190

tcc tca tta acc cgt gca gaa cgg gtg ggg ggc tat tgt gca act cta 624
Ser Ser Leu Thr Arg Ala Glu Arg Val Gly Gly Tyr Cys Ala Thr Leu
        195                 200                 205

cta aaa ttt ggc ctg ccg ttt cac agc gat tgg gtg ttg gag tgc act 672
Leu Lys Phe Gly Leu Pro Phe His Ser Asp Trp Val Leu Glu Cys Thr
    210                 215                 220

tcc agc cag aag caa gcc gcg gaa gct atc acg gcg ctt tta cgt cat 720
Ser Ser Gln Lys Gln Ala Ala Glu Ala Ile Thr Ala Leu Leu Arg His
225                 230                 235                 240

aac ccg acc atc agt gcc gtg gtt tgc tat aac gaa act att gcg atg 768
Asn Pro Thr Ile Ser Ala Val Val Cys Tyr Asn Glu Thr Ile Ala Met
                245                 250                 255

ggg gca tgg ttt ggt ttg ctg aaa gca ggg cgg caa agc ggg gaa agc 816
Gly Ala Trp Phe Gly Leu Leu Lys Ala Gly Arg Gln Ser Gly Glu Ser
            260                 265                 270

gga gtc gat cgt tac ttt gag caa cag gtt tcg ctg gcg gca ttt acc 864
Gly Val Asp Arg Tyr Phe Glu Gln Gln Val Ser Leu Ala Ala Phe Thr
        275                 280                 285

gat gcg aca cca acc aca ctt gat gat ata cct gtt acc tgg gcc agc 912
Asp Ala Thr Pro Thr Thr Leu Asp Asp Ile Pro Val Thr Trp Ala Ser
    290                 295                 300

acg cca gcg cgg gaa ctt ggt atc aca ctt gcg gat cgc atg atg caa 960
Thr Pro Ala Arg Glu Leu Gly Ile Thr Leu Ala Asp Arg Met Met Gln
305                 310                 315                 320

aaa atc acc cat gaa gag acg cat tca cgc aat ctt att att ccc gcc 1008
Lys Ile Thr His Glu Glu Thr His Ser Arg Asn Leu Ile Ile Pro Ala
                325                 330                 335

cgg ctc att gcg gcg aaa taa 1029
Arg Leu Ile Ala Ala Lys
            340

<210> 4
<211> 342
<212> PRT
<213> Shigella flexneri

<400> 4

Met Ala Thr Ala Lys Lys Ile Thr Ile His Asp Val Ala Leu Ala Ala
1               5                   10                  15

Gly Val Ser Val Ser Thr Val Ser Leu Val Leu Asn Gly Lys Gly Arg
            20                  25                  30

Ile Ser Thr Ala Thr Gly Glu Arg Val Asn Ala Ala Ile Glu Glu Leu
        35                  40                  45

Gly Phe Val Arg Asn Arg Gln Ala Ser Ala Leu Arg Gly Gly Gln Ser

50                  55                  60

Gly Val Ile Gly Leu Ile Val Arg Asp Leu Ser Ala Pro Phe Tyr Ala
65                  70                  75                  80

Glu Leu Thr Ala Gly Leu Thr Glu Ala Leu Glu Ala Gln Gly Arg Met
                85                  90                  95

Val Phe Leu Leu His Gly Gly Lys Asp Gly Glu Gln Leu Ala Gln Arg
            100                 105                 110

Phe Ser Leu Leu Leu Asn Gln Gly Val Asp Gly Val Val Ile Ala Gly
        115                 120                 125

Ala Ala Gly Ser Ser Asp Asp Leu Arg Arg Met Ala Glu Glu Lys Ala
    130                 135                 140

Ile Pro Val Ile Phe Ala Ser Arg Ala Ser Tyr Leu Asp Asp Val Asp
145                 150                 155                 160

Thr Val Arg Pro Asp Asn Met Gln Ala Ala Gln Leu Leu Thr Glu His
                165                 170                 175

Leu Ile Arg Asn Gly His Gln Arg Ile Ala Trp Leu Gly Gly Gln Ser
            180                 185                 190

Ser Ser Leu Thr Arg Ala Glu Arg Val Gly Gly Tyr Cys Ala Thr Leu
        195                 200                 205

Leu Lys Phe Gly Leu Pro Phe His Ser Asp Trp Val Leu Glu Cys Thr
    210                 215                 220

Ser Ser Gln Lys Gln Ala Ala Glu Ala Ile Thr Ala Leu Leu Arg His
225                 230                 235                 240

Asn Pro Thr Ile Ser Ala Val Val Cys Tyr Asn Glu Thr Ile Ala Met
                245                 250                 255

Gly Ala Trp Phe Gly Leu Leu Lys Ala Gly Arg Gln Ser Gly Glu Ser
            260                 265                 270

Gly Val Asp Arg Tyr Phe Glu Gln Gln Val Ser Leu Ala Ala Phe Thr
        275                 280                 285

Asp Ala Thr Pro Thr Thr Leu Asp Asp Ile Pro Val Thr Trp Ala Ser
    290                 295                 300

Thr Pro Ala Arg Glu Leu Gly Ile Thr Leu Ala Asp Arg Met Met Gln
305                 310                 315                 320

Lys Ile Thr His Glu Glu Thr His Ser Arg Asn Leu Ile Ile Pro Ala
                325                 330                 335

Arg Leu Ile Ala Ala Lys
            340

<210>  5
<211>  31
<212>  DNA
<213>  Escherichia coli

<220>
<221>  misc_feature
<222>  (1)..(31)
<223>  Primer malI1

<400>  5
cgttttatct agaacataat cagggagtag g                                    31

<210>  6
<211>  29
<212>  DNA
<213>  Escherichia coli

<220>
<221>  misc_feature
<222>  (1)..(29)
<223>  Primer malI2

<400>  6
cactaccagg tatagaagct taagagagg                                       29

<210>  7
<211>  1098
<212>  DNA
<213>  Escherichia coli

<220>
<221>  misc_feature
<222>  (1)..(31)
<223>  Primer-Bindestelle malI1

<220>
<221>  CDS
<222>  (40)..(1068)
<223>  malI-Kodierregion

<220>
<221>  misc_feature
<222>  (1070)..(1098)
<223>  Primer-Bindestelle malI2

<400>  7
cgttttatct agaacataat cagggagtag gtcatctgc atg gct acc gcc aaa        54
                                           Met Ala Thr Ala Lys
                                           1               5

aaa ata acc att cat gat gtt gcg ctg gct gcg ggc gtg tcg gta agt      102

Lys Ile Thr Ile His Asp Val Ala Leu Ala Ala Gly Val Ser Val Ser
                10                  15                  20

acc gtt tcg ctg gtg ctt agt ggc aaa ggg cga atc tct acc gcc aca      150
Thr Val Ser Leu Val Leu Ser Gly Lys Gly Arg Ile Ser Thr Ala Thr
            25                  30                  35

gga gaa cgc gtt aac gcc gcc att gaa gag ctg gga ttt gtg cgc aat      198
Gly Glu Arg Val Asn Ala Ala Ile Glu Glu Leu Gly Phe Val Arg Asn
        40                  45                  50

cgc cag gcg tcg gcg ctg cgc ggc ggg caa agc ggc gtc att ggt ttg      246
Arg Gln Ala Ser Ala Leu Arg Gly Gly Gln Ser Gly Val Ile Gly Leu
    55                  60                  65

atc gtc cgt gat tta tct gcg ccg ttt tac gcc gaa ttg acg gcc gga      294
Ile Val Arg Asp Leu Ser Ala Pro Phe Tyr Ala Glu Leu Thr Ala Gly
70                  75                  80                  85

ttg acg gaa gct ctg gaa gcg cag gga cgg atg gtt ttt ttg ctt cac      342
Leu Thr Glu Ala Leu Glu Ala Gln Gly Arg Met Val Phe Leu Leu His
                90                  95                  100

ggc ggt aaa gac ggt gag cag ctg gca cag cgg ttt tca ctg tta ctg      390
Gly Gly Lys Asp Gly Glu Gln Leu Ala Gln Arg Phe Ser Leu Leu Leu
            105                 110                 115

aat cag ggt gtc gat ggt gtg gta att gcc gga gct gca gga agt agc      438
Asn Gln Gly Val Asp Gly Val Val Ile Ala Gly Ala Ala Gly Ser Ser
        120                 125                 130

gat gac ctg cga cgg atg gca gaa gaa aaa gct atc ccg gtg att ttc      486
Asp Asp Leu Arg Arg Met Ala Glu Glu Lys Ala Ile Pro Val Ile Phe
    135                 140                 145

gct tcc cgt gcc agt tat ctt gat gat gtt gat acg gtt cgc ccg gac      534
Ala Ser Arg Ala Ser Tyr Leu Asp Asp Val Asp Thr Val Arg Pro Asp
150                 155                 160                 165

aac atg cag gct gca cag ttg ttg acg gag cat ctc att cgc aat ggg      582
Asn Met Gln Ala Ala Gln Leu Leu Thr Glu His Leu Ile Arg Asn Gly
                170                 175                 180

cat cag cgg atc gcc tgg ctg gga ggg caa agt tcc tca tta acc cgt      630
His Gln Arg Ile Ala Trp Leu Gly Gly Gln Ser Ser Ser Leu Thr Arg
            185                 190                 195

gca gaa cgg gtt ggg ggc tat tgt gca act cta cta aaa ttt ggc ctg      678
Ala Glu Arg Val Gly Gly Tyr Cys Ala Thr Leu Leu Lys Phe Gly Leu
        200                 205                 210

ccg ttt cat agc gat tgg gtg ttg gag tgc act tcc agc cag aag caa      726
Pro Phe His Ser Asp Trp Val Leu Glu Cys Thr Ser Ser Gln Lys Gln
    215                 220                 225

gcc gcg gaa gct atc acg gcg ctt tta cgt cat aac ccg acc atc agt      774
Ala Ala Glu Ala Ile Thr Ala Leu Leu Arg His Asn Pro Thr Ile Ser
230                 235                 240                 245

gcc gtg gtt tgc tat aac gaa act att gcg atg ggg gca tgg ttt ggt      822
Ala Val Val Cys Tyr Asn Glu Thr Ile Ala Met Gly Ala Trp Phe Gly
                250                 255                 260

ttg ctc aaa gca ggg cgg caa agc ggg gaa agc gga gtc gat cgt tac      870
Leu Leu Lys Ala Gly Arg Gln Ser Gly Glu Ser Gly Val Asp Arg Tyr
            265                 270                 275

ttt gag caa cag gtt tcg ctg gcg gca ttt acc gat gcg aca cca acc       918
Phe Glu Gln Gln Val Ser Leu Ala Ala Phe Thr Asp Ala Thr Pro Thr
        280                 285                 290

aca ctt gat gat ata cct gtt acc tgg gcc agc acg cca gcg cgg gaa       966
Thr Leu Asp Asp Ile Pro Val Thr Trp Ala Ser Thr Pro Ala Arg Glu
    295                 300                 305

ctt ggt atc aca ctt gcg gat cgc atg atg caa aaa atc acc cat gaa      1014
Leu Gly Ile Thr Leu Ala Asp Arg Met Met Gln Lys Ile Thr His Glu
310                 315                 320                 325

gag acg cat tca cgc aat ctt att att ccc gcc cgg ctc att gca gcg      1062
Glu Thr His Ser Arg Asn Leu Ile Ile Pro Ala Arg Leu Ile Ala Ala
                330                 335                 340

aaa taa tcctctctta agcttctata cctggtagtg                             1098
Lys

<210>  8
<211>  342
<212>  PRT
<213>  Escherichia coli

<400>  8

Met Ala Thr Ala Lys Lys Ile Thr Ile His Asp Val Ala Leu Ala Ala
1               5                   10                  15

Gly Val Ser Val Ser Thr Val Ser Leu Val Leu Ser Gly Lys Gly Arg
            20                  25                  30

Ile Ser Thr Ala Thr Gly Glu Arg Val Asn Ala Ala Ile Glu Glu Leu
        35                  40                  45

Gly Phe Val Arg Asn Arg Gln Ala Ser Ala Leu Arg Gly Gly Gln Ser
    50                  55                  60

Gly Val Ile Gly Leu Ile Val Arg Asp Leu Ser Ala Pro Phe Tyr Ala
65                  70                  75                  80

Glu Leu Thr Ala Gly Leu Thr Glu Ala Leu Glu Ala Gln Gly Arg Met
                85                  90                  95

Val Phe Leu Leu His Gly Gly Lys Asp Gly Glu Gln Leu Ala Gln Arg
            100                 105                 110

Phe Ser Leu Leu Leu Asn Gln Gly Val Asp Gly Val Val Ile Ala Gly
        115                 120                 125

Ala Ala Gly Ser Ser Asp Asp Leu Arg Arg Met Ala Glu Glu Lys Ala
    130                 135                 140

Ile Pro Val Ile Phe Ala Ser Arg Ala Ser Tyr Leu Asp Asp Val Asp

145                 150                 155                 160

Thr Val Arg Pro Asp Asn Met Gln Ala Ala Gln Leu Leu Thr Glu His
                165                 170                 175

Leu Ile Arg Asn Gly His Gln Arg Ile Ala Trp Leu Gly Gly Gln Ser
            180                 185                 190

Ser Ser Leu Thr Arg Ala Glu Arg Val Gly Gly Tyr Cys Ala Thr Leu
        195                 200                 205

Leu Lys Phe Gly Leu Pro Phe His Ser Asp Trp Val Leu Glu Cys Thr
    210                 215                 220

Ser Ser Gln Lys Gln Ala Ala Glu Ala Ile Thr Ala Leu Leu Arg His
225                 230                 235                 240

Asn Pro Thr Ile Ser Ala Val Val Cys Tyr Asn Glu Thr Ile Ala Met
                245                 250                 255

Gly Ala Trp Phe Gly Leu Leu Lys Ala Gly Arg Gln Ser Gly Glu Ser
            260                 265                 270

Gly Val Asp Arg Tyr Phe Glu Gln Gln Val Ser Leu Ala Ala Phe Thr
        275                 280                 285

Asp Ala Thr Pro Thr Thr Leu Asp Asp Ile Pro Val Thr Trp Ala Ser
    290                 295                 300

Thr Pro Ala Arg Glu Leu Gly Ile Thr Leu Ala Asp Arg Met Met Gln
305                 310                 315                 320

Lys Ile Thr His Glu Glu Thr His Ser Arg Asn Leu Ile Ile Pro Ala
                325                 330                 335

Arg Leu Ile Ala Ala Lys
            340

<210>  9
<211>  1035
<212>  DNA
<213>  Escherichia coli

<220>
<221>  CDS
<222>  (1)..(1035)
<223>  yibD-Kodierregion

<400>  9
atg atg aac agc acc aat aaa ctt agt gtt att att ccg tta tat aat        48
Met Met Asn Ser Thr Asn Lys Leu Ser Val Ile Ile Pro Leu Tyr Asn
1               5                   10                  15

gcg ggc gat gat ttc cgc act tgt atg gaa tct tta att acg caa acc 96
Ala Gly Asp Asp Phe Arg Thr Cys Met Glu Ser Leu Ile Thr Gln Thr
            20                  25                  30

tgg act gct ctg gaa atc att att att aac gat ggt tca acg gat aat 144
Trp Thr Ala Leu Glu Ile Ile Ile Ile Asn Asp Gly Ser Thr Asp Asn
        35                  40                  45

tct gtt gaa ata gca aag tat tac gca gaa aac tat ccg cac gtt cgt 192
Ser Val Glu Ile Ala Lys Tyr Tyr Ala Glu Asn Tyr Pro His Val Arg
    50                  55                  60

ttg ttg cat cag gcg aat gct ggc gca tcg gtg gcg cgt aat cgt ggg 240
Leu Leu His Gln Ala Asn Ala Gly Ala Ser Val Ala Arg Asn Arg Gly
65                  70                  75                  80

att gaa gtg gca acg ggc aaa tat gtc gct ttt gtc gat gct gac gat 288
Ile Glu Val Ala Thr Gly Lys Tyr Val Ala Phe Val Asp Ala Asp Asp
                85                  90                  95

gaa gtc tat ccc acc atg tac gaa acg ctg atg acc atg gcg tta gag 336
Glu Val Tyr Pro Thr Met Tyr Glu Thr Leu Met Thr Met Ala Leu Glu
            100                 105                 110

gac gac ctc gac gtg gcg cag tgc aac gct gac tgg tgt ttt cgt gaa 384
Asp Asp Leu Asp Val Ala Gln Cys Asn Ala Asp Trp Cys Phe Arg Glu
        115                 120                 125

acg gga gaa acc tgg caa tcc atc ccc acc gat cgc ctt cgc tca acc 432
Thr Gly Glu Thr Trp Gln Ser Ile Pro Thr Asp Arg Leu Arg Ser Thr
    130                 135                 140

ggc gta tta acc ggc ccg gac tgg ctg cgg atg ggg ctt tct tcg cgc 480
Gly Val Leu Thr Gly Pro Asp Trp Leu Arg Met Gly Leu Ser Ser Arg
145                 150                 155                 160

cgt tgg act cac gtt gtc tgg atg ggg gtt tat cgc cgt gat gtt att 528
Arg Trp Thr His Val Val Trp Met Gly Val Tyr Arg Arg Asp Val Ile
                165                 170                 175

gtt aaa aat aac att aaa ttt att gcc gga tta cat cat cag gat att 576
Val Lys Asn Asn Ile Lys Phe Ile Ala Gly Leu His His Gln Asp Ile
            180                 185                 190

gtc tgg aca aca gaa ttc atg ttt aac gcg ctg cgt gcg cga tat acc 624
Val Trp Thr Thr Glu Phe Met Phe Asn Ala Leu Arg Ala Arg Tyr Thr
        195                 200                 205

gag caa tca tta tat aaa tat tat ctg cat aat acg tca gtg agt cgg 672
Glu Gln Ser Leu Tyr Lys Tyr Tyr Leu His Asn Thr Ser Val Ser Arg
    210                 215                 220

ttg cat aga caa ggg aat aaa aac ctt aat tat caa cgt cac tat att 720
Leu His Arg Gln Gly Asn Lys Asn Leu Asn Tyr Gln Arg His Tyr Ile
225                 230                 235                 240

aag att acc cgc ctg ctg gag aaa tta aat cga aat tat gcc gac aaa 768
Lys Ile Thr Arg Leu Leu Glu Lys Leu Asn Arg Asn Tyr Ala Asp Lys
                245                 250                 255

att atg att tat ccg gaa ttt cat cag caa ata act tac gag gca ttg 816
Ile Met Ile Tyr Pro Glu Phe His Gln Gln Ile Thr Tyr Glu Ala Leu
            260                 265                 270

cgt gtt tgc cat gcg gtg cgc aaa gag ccg gat att ctt acc cgc caa 864

Arg Val Cys His Ala Val Arg Lys Glu Pro Asp Ile Leu Thr Arg Gln
        275                 280                 285

cgg atg att gcc gag ata ttt act tcc ggt atg tat aag cgc ctg att       912
Arg Met Ile Ala Glu Ile Phe Thr Ser Gly Met Tyr Lys Arg Leu Ile
    290                 295                 300

acc aat gtg cgc agc gtg aag gtc ggt tac cag gcg tta ctg tgg tct       960
Thr Asn Val Arg Ser Val Lys Val Gly Tyr Gln Ala Leu Leu Trp Ser
305                 310                 315                 320

ttc cgc tta tgg caa tgg cgc gac aaa acg cgg tcg cac cat cgc att      1008
Phe Arg Leu Trp Gln Trp Arg Asp Lys Thr Arg Ser His His Arg Ile
                325                 330                 335

acg cgt agc gcc ttt aat ttg cgc tag                                  1035
Thr Arg Ser Ala Phe Asn Leu Arg
            340

<210>  10
<211>  344
<212>  PRT
<213>  Escherichia coli

<400>  10

Met Met Asn Ser Thr Asn Lys Leu Ser Val Ile Ile Pro Leu Tyr Asn
1               5                   10                  15

Ala Gly Asp Asp Phe Arg Thr Cys Met Glu Ser Leu Ile Thr Gln Thr
            20                  25                  30

Trp Thr Ala Leu Glu Ile Ile Ile Ile Asn Asp Gly Ser Thr Asp Asn
        35                  40                  45

Ser Val Glu Ile Ala Lys Tyr Tyr Ala Glu Asn Tyr Pro His Val Arg
    50                  55                  60

Leu Leu His Gln Ala Asn Ala Gly Ala Ser Val Ala Arg Asn Arg Gly
65                  70                  75                  80

Ile Glu Val Ala Thr Gly Lys Tyr Val Ala Phe Val Asp Ala Asp Asp
                85                  90                  95

Glu Val Tyr Pro Thr Met Tyr Glu Thr Leu Met Thr Met Ala Leu Glu
            100                 105                 110

Asp Asp Leu Asp Val Ala Gln Cys Asn Ala Asp Trp Cys Phe Arg Glu
        115                 120                 125

Thr Gly Glu Thr Trp Gln Ser Ile Pro Thr Asp Arg Leu Arg Ser Thr
    130                 135                 140

Gly Val Leu Thr Gly Pro Asp Trp Leu Arg Met Gly Leu Ser Ser Arg
145                 150                 155                 160

Arg Trp Thr His Val Val Trp Met Gly Val Tyr Arg Arg Asp Val Ile
                165                 170                 175

Val Lys Asn Asn Ile Lys Phe Ile Ala Gly Leu His His Gln Asp Ile
            180                 185                 190

Val Trp Thr Thr Glu Phe Met Phe Asn Ala Leu Arg Ala Arg Tyr Thr
        195                 200                 205

Glu Gln Ser Leu Tyr Lys Tyr Tyr Leu His Asn Thr Ser Val Ser Arg
    210                 215                 220

Leu His Arg Gln Gly Asn Lys Asn Leu Asn Tyr Gln Arg His Tyr Ile
225                 230                 235                 240

Lys Ile Thr Arg Leu Leu Glu Lys Leu Asn Arg Asn Tyr Ala Asp Lys
                245                 250                 255

Ile Met Ile Tyr Pro Glu Phe His Gln Gln Ile Thr Tyr Glu Ala Leu
            260                 265                 270

Arg Val Cys His Ala Val Arg Lys Glu Pro Asp Ile Leu Thr Arg Gln
        275                 280                 285

Arg Met Ile Ala Glu Ile Phe Thr Ser Gly Met Tyr Lys Arg Leu Ile
    290                 295                 300

Thr Asn Val Arg Ser Val Lys Val Gly Tyr Gln Ala Leu Leu Trp Ser
305                 310                 315                 320

Phe Arg Leu Trp Gln Trp Arg Asp Lys Thr Arg Ser His His Arg Ile
                325                 330                 335

Thr Arg Ser Ala Phe Asn Leu Arg
            340

<210>  11
<211>  1332
<212>  DNA
<213>  Escherichia coli

<220>
<221>  CDS
<222>  (1)..(1332)
<223>  yaaU-Kodierregion

<400>  11
atg caa ccg tcc aga aac ttt gac gat ctc aaa ttc tcc tct att cac        48
Met Gln Pro Ser Arg Asn Phe Asp Asp Leu Lys Phe Ser Ser Ile His
1               5                   10                  15

cgc cgc att ttg ctg tgg gga agc ggt ggt ccg ttt ctg gat ggt tat        96

Arg Arg Ile Leu Leu Trp Gly Ser Gly Gly Pro Phe Leu Asp Gly Tyr
            20                  25                  30

gta ctg gta atg att ggc gtg gcg ctg gag caa ctg acg ccg gcg ctg      144
Val Leu Val Met Ile Gly Val Ala Leu Glu Gln Leu Thr Pro Ala Leu
        35                  40                  45

aaa ctg gac gct gac tgg att ggc ttg ctg ggc gcg gga acg ctc gcc      192
Lys Leu Asp Ala Asp Trp Ile Gly Leu Leu Gly Ala Gly Thr Leu Ala
    50                  55                  60

ggg ctg ttc gtt ggc aca tcg ctg ttt ggt tat att tcc gat aaa gtc      240
Gly Leu Phe Val Gly Thr Ser Leu Phe Gly Tyr Ile Ser Asp Lys Val
65                  70                  75                  80

gga cgg cgc aaa atg ttc ctc att gat atc atc gcc atc ggc gtg ata      288
Gly Arg Arg Lys Met Phe Leu Ile Asp Ile Ile Ala Ile Gly Val Ile
                85                  90                  95

tcg gtg gcg acg atg ttt gtt tca tcc ccc gtc gaa ctg ttg gtg atg      336
Ser Val Ala Thr Met Phe Val Ser Ser Pro Val Glu Leu Leu Val Met
            100                 105                 110

cgg gta ctt atc ggc att gtc atc ggt gca gat tat ccc atc gcc acc      384
Arg Val Leu Ile Gly Ile Val Ile Gly Ala Asp Tyr Pro Ile Ala Thr
        115                 120                 125

tca atg atc acc gag ttc tcc agt acc cgt cag cgg gcg ttt tcc atc      432
Ser Met Ile Thr Glu Phe Ser Ser Thr Arg Gln Arg Ala Phe Ser Ile
    130                 135                 140

agc ttt att gcc gcg atg tgg tat gtc ggc gcg acc tgt gcc gat ctg      480
Ser Phe Ile Ala Ala Met Trp Tyr Val Gly Ala Thr Cys Ala Asp Leu
145                 150                 155                 160

gtc ggc tac tgg ctt tat gat gtg gaa ggc ggc tgg cgc tgg atg ctg      528
Val Gly Tyr Trp Leu Tyr Asp Val Glu Gly Gly Trp Arg Trp Met Leu
                165                 170                 175

ggt agc gcg gcg atc ccc tgt ttg ttg att ttg att ggt cga ttc gaa      576
Gly Ser Ala Ala Ile Pro Cys Leu Leu Ile Leu Ile Gly Arg Phe Glu
            180                 185                 190

ctg cct gaa tct ccc cgc tgg tta tta cgc aaa ggg cga gta aaa gag      624
Leu Pro Glu Ser Pro Arg Trp Leu Leu Arg Lys Gly Arg Val Lys Glu
        195                 200                 205

tgc gaa gag atg atg atc aaa ctg ttt ggc gaa ccg gtg gct ttc gat      672
Cys Glu Glu Met Met Ile Lys Leu Phe Gly Glu Pro Val Ala Phe Asp
    210                 215                 220

gaa gag cag ccg cag caa acc cgt ttt cgc gat ctg ttt aat cgc cgc      720
Glu Glu Gln Pro Gln Gln Thr Arg Phe Arg Asp Leu Phe Asn Arg Arg
225                 230                 235                 240

cat ttt cct ttt gtt ctg ttt gtt gcc gcc atc tgg acc tgc cag gtg      768
His Phe Pro Phe Val Leu Phe Val Ala Ala Ile Trp Thr Cys Gln Val
                245                 250                 255

atc cca atg ttc gcc att tac acc ttt ggc ccg caa atc gtt ggt ttg      816
Ile Pro Met Phe Ala Ile Tyr Thr Phe Gly Pro Gln Ile Val Gly Leu
            260                 265                 270

ttg gga ttg ggg gtt ggc aaa aac gcg gca cta ggg aat gtg gtg att      864
Leu Gly Leu Gly Val Gly Lys Asn Ala Ala Leu Gly Asn Val Val Ile
        275                 280                 285

agc ctg ttc ttt atg ctc ggc tgt att ccg ccg atg ctg tgg tta aac        912
Ser Leu Phe Phe Met Leu Gly Cys Ile Pro Pro Met Leu Trp Leu Asn
    290                 295                 300

act gcc gga cgg cgt cca ttg ttg att ggc agc ttt gcc atg atg acg        960
Thr Ala Gly Arg Arg Pro Leu Leu Ile Gly Ser Phe Ala Met Met Thr
305                 310                 315                 320

ctg gcg ctg gcg gtt ttg ggg cta atc ccg gat atg ggg atc tgg ctg       1008
Leu Ala Leu Ala Val Leu Gly Leu Ile Pro Asp Met Gly Ile Trp Leu
                325                 330                 335

gta gtg atg gcc ttt gcg gtg tat gcc ttt ttc tct ggc ggg ccg ggt       1056
Val Val Met Ala Phe Ala Val Tyr Ala Phe Phe Ser Gly Gly Pro Gly
            340                 345                 350

aat ttg cag tgg ctc tat cct aat gaa ctc ttc ccg aca gat atc cgc       1104
Asn Leu Gln Trp Leu Tyr Pro Asn Glu Leu Phe Pro Thr Asp Ile Arg
        355                 360                 365

gcc tct gcc gtg ggc gtg att atg tcc tta agt cgt att ggc acc att       1152
Ala Ser Ala Val Gly Val Ile Met Ser Leu Ser Arg Ile Gly Thr Ile
    370                 375                 380

gtt tcg acc tgg gca cta ccg atc ttt atc aat aat tac ggt atc agt       1200
Val Ser Thr Trp Ala Leu Pro Ile Phe Ile Asn Asn Tyr Gly Ile Ser
385                 390                 395                 400

aac acg atg cta atg ggg gcg ggt atc tcg ctg ttt ggc ttg ttg att       1248
Asn Thr Met Leu Met Gly Ala Gly Ile Ser Leu Phe Gly Leu Leu Ile
                405                 410                 415

tcc gta gcg ttt gcc ccg gag act cga ggg atg tca ctg gcg cag acc       1296
Ser Val Ala Phe Ala Pro Glu Thr Arg Gly Met Ser Leu Ala Gln Thr
            420                 425                 430

agc aat atg acg atc cgc ggg cag aga atg ggg taa                      1332
Ser Asn Met Thr Ile Arg Gly Gln Arg Met Gly
        435                 440


<210>  12
<211>  443
<212>  PRT
<213>  Escherichia coli

<400>  12

Met Gln Pro Ser Arg Asn Phe Asp Asp Leu Lys Phe Ser Ser Ile His
1               5                   10                  15


Arg Arg Ile Leu Leu Trp Gly Ser Gly Gly Pro Phe Leu Asp Gly Tyr
            20                  25                  30


Val Leu Val Met Ile Gly Val Ala Leu Glu Gln Leu Thr Pro Ala Leu
        35                  40                  45


Lys Leu Asp Ala Asp Trp Ile Gly Leu Leu Gly Ala Gly Thr Leu Ala
    50                  55                  60


Gly Leu Phe Val Gly Thr Ser Leu Phe Gly Tyr Ile Ser Asp Lys Val

65                  70                  75                  80

Gly Arg Arg Lys Met Phe Leu Ile Asp Ile Ile Ala Ile Gly Val Ile
                85                  90                  95

Ser Val Ala Thr Met Phe Val Ser Ser Pro Val Glu Leu Leu Val Met
            100                 105                 110

Arg Val Leu Ile Gly Ile Val Ile Gly Ala Asp Tyr Pro Ile Ala Thr
        115                 120                 125

Ser Met Ile Thr Glu Phe Ser Ser Thr Arg Gln Arg Ala Phe Ser Ile
    130                 135                 140

Ser Phe Ile Ala Ala Met Trp Tyr Val Gly Ala Thr Cys Ala Asp Leu
145                 150                 155                 160

Val Gly Tyr Trp Leu Tyr Asp Val Glu Gly Gly Trp Arg Trp Met Leu
                165                 170                 175

Gly Ser Ala Ala Ile Pro Cys Leu Leu Ile Leu Ile Gly Arg Phe Glu
            180                 185                 190

Leu Pro Glu Ser Pro Arg Trp Leu Leu Arg Lys Gly Arg Val Lys Glu
        195                 200                 205

Cys Glu Glu Met Met Ile Lys Leu Phe Gly Glu Pro Val Ala Phe Asp
    210                 215                 220

Glu Glu Gln Pro Gln Gln Thr Arg Phe Arg Asp Leu Phe Asn Arg Arg
225                 230                 235                 240

His Phe Pro Phe Val Leu Phe Val Ala Ala Ile Trp Thr Cys Gln Val
                245                 250                 255

Ile Pro Met Phe Ala Ile Tyr Thr Phe Gly Pro Gln Ile Val Gly Leu
            260                 265                 270

Leu Gly Leu Gly Val Gly Lys Asn Ala Ala Leu Gly Asn Val Val Ile
        275                 280                 285

Ser Leu Phe Phe Met Leu Gly Cys Ile Pro Pro Met Leu Trp Leu Asn
    290                 295                 300

Thr Ala Gly Arg Arg Pro Leu Leu Ile Gly Ser Phe Ala Met Met Thr
305                 310                 315                 320

Leu Ala Leu Ala Val Leu Gly Leu Ile Pro Asp Met Gly Ile Trp Leu
                325                 330                 335

Val Val Met Ala Phe Ala Val Tyr Ala Phe Phe Ser Gly Gly Pro Gly
            340                 345                 350

Asn Leu Gln Trp Leu Tyr Pro Asn Glu Leu Phe Pro Thr Asp Ile Arg
        355                 360                 365

Ala Ser Ala Val Gly Val Ile Met Ser Leu Ser Arg Ile Gly Thr Ile
    370                 375                 380

Val Ser Thr Trp Ala Leu Pro Ile Phe Ile Asn Asn Tyr Gly Ile Ser
385                 390                 395                 400

Asn Thr Met Leu Met Gly Ala Gly Ile Ser Leu Phe Gly Leu Leu Ile
                405                 410                 415

Ser Val Ala Phe Ala Pro Glu Thr Arg Gly Met Ser Leu Ala Gln Thr
            420                 425                 430

Ser Asn Met Thr Ile Arg Gly Gln Arg Met Gly
        435                 440

<210>  13
<211>  882
<212>  DNA
<213>  Escherichia coli

<220>
<221>  CDS
<222>  (1)..(882)
<223>  yddG-Kodierregion

<400>  13
atg aca cga caa aaa gca acg ctc ata ggg ctg ata gcg atc gtc ctg       48
Met Thr Arg Gln Lys Ala Thr Leu Ile Gly Leu Ile Ala Ile Val Leu
1               5                   10                  15

tgg agc acg atg gta gga ttg att cgc ggt gtc agt gag ggg ctc ggc       96
Trp Ser Thr Met Val Gly Leu Ile Arg Gly Val Ser Glu Gly Leu Gly
            20                  25                  30

ccg gtc ggc ggc gca gct gct atc tat tca tta agc ggg ctg ctg tta      144
Pro Val Gly Gly Ala Ala Ala Ile Tyr Ser Leu Ser Gly Leu Leu Leu
        35                  40                  45

atc ttc acg gtt gga ttt ccg cgt att cgg caa atc ccg aaa ggc tat      192
Ile Phe Thr Val Gly Phe Pro Arg Ile Arg Gln Ile Pro Lys Gly Tyr
    50                  55                  60

tta ctc gcc ggg agt ctg tta ttc gtc agc tat gaa atc tgt ctg gcg      240
Leu Leu Ala Gly Ser Leu Leu Phe Val Ser Tyr Glu Ile Cys Leu Ala
65                  70                  75                  80

ctt tcc tta ggg tat gcg gcg acc cat cat cag gcg att gaa gtg ggt      288
Leu Ser Leu Gly Tyr Ala Ala Thr His His Gln Ala Ile Glu Val Gly
                85                  90                  95

atg gtg aac tat ctg tgg ccc agc ctg aca att ctc ttt gcc att ctg      336

Met Val Asn Tyr Leu Trp Pro Ser Leu Thr Ile Leu Phe Ala Ile Leu
            100                 105                 110

ttt aat ggt cag aaa acc aac tgg ttg att gta cct gga tta tta tta      384
Phe Asn Gly Gln Lys Thr Asn Trp Leu Ile Val Pro Gly Leu Leu Leu
        115                 120                 125

gcc ctc gtc ggc gtc tgt tgg gtg tta ggc ggt gac aat ggg tta cat      432
Ala Leu Val Gly Val Cys Trp Val Leu Gly Gly Asp Asn Gly Leu His
    130                 135                 140

tat gat gaa atc atc aat aat atc acc acc agc cca ttg agt tat ttc      480
Tyr Asp Glu Ile Ile Asn Asn Ile Thr Thr Ser Pro Leu Ser Tyr Phe
145                 150                 155                 160

ctg gcg ttc att ggt gcg ttt atc tgg gca gcc tat tgc aca gta acg      528
Leu Ala Phe Ile Gly Ala Phe Ile Trp Ala Ala Tyr Cys Thr Val Thr
                165                 170                 175

aat aaa tac gca cgc gga ttt aat gga att acc gtt ttt gtc ctg cta      576
Asn Lys Tyr Ala Arg Gly Phe Asn Gly Ile Thr Val Phe Val Leu Leu
            180                 185                 190

acg gga gca agt ctg tgg gtt tac tat ttt ctt acg cca caa cca gaa      624
Thr Gly Ala Ser Leu Trp Val Tyr Tyr Phe Leu Thr Pro Gln Pro Glu
        195                 200                 205

atg ata ttt agc acg ccc gtc atg att aaa ctc atc tct gcg gca ttt      672
Met Ile Phe Ser Thr Pro Val Met Ile Lys Leu Ile Ser Ala Ala Phe
    210                 215                 220

acc tta gga ttt gct tat gct gca tgg aat gtc ggt ata ttg cat ggc      720
Thr Leu Gly Phe Ala Tyr Ala Ala Trp Asn Val Gly Ile Leu His Gly
225                 230                 235                 240

aat gtc acc att atg gcg gta ggt tcg tat ttt acg cct gta ctt tcc      768
Asn Val Thr Ile Met Ala Val Gly Ser Tyr Phe Thr Pro Val Leu Ser
                245                 250                 255

tca gcg ctt gca gcc gtg ctg ctc agc gcc ccg ctg tcg ttc tcg ttc      816
Ser Ala Leu Ala Ala Val Leu Leu Ser Ala Pro Leu Ser Phe Ser Phe
            260                 265                 270

tgg caa ggc gcg ctg atg gtc tgc ggc ggt tcc ctg ctc tgc tgg ctg      864
Trp Gln Gly Ala Leu Met Val Cys Gly Gly Ser Leu Leu Cys Trp Leu
        275                 280                 285

gcg aca cgt cgt ggt taa                                              882
Ala Thr Arg Arg Gly
    290

<210>  14
<211>  293
<212>  PRT
<213>  Escherichia coli

<400>  14

Met Thr Arg Gln Lys Ala Thr Leu Ile Gly Leu Ile Ala Ile Val Leu
1               5                   10                  15

Trp Ser Thr Met Val Gly Leu Ile Arg Gly Val Ser Glu Gly Leu Gly
            20                  25                  30

Pro Val Gly Gly Ala Ala Ala Ile Tyr Ser Leu Ser Gly Leu Leu Leu
        35                  40                  45

Ile Phe Thr Val Gly Phe Pro Arg Ile Arg Gln Ile Pro Lys Gly Tyr
    50                  55                  60

Leu Leu Ala Gly Ser Leu Leu Phe Val Ser Tyr Glu Ile Cys Leu Ala
65                  70                  75                  80

Leu Ser Leu Gly Tyr Ala Ala Thr His His Gln Ala Ile Glu Val Gly
                85                  90                  95

Met Val Asn Tyr Leu Trp Pro Ser Leu Thr Ile Leu Phe Ala Ile Leu
            100                 105                 110

Phe Asn Gly Gln Lys Thr Asn Trp Leu Ile Val Pro Gly Leu Leu Leu
        115                 120                 125

Ala Leu Val Gly Val Cys Trp Val Leu Gly Gly Asp Asn Gly Leu His
    130                 135                 140

Tyr Asp Glu Ile Ile Asn Asn Ile Thr Thr Ser Pro Leu Ser Tyr Phe
145                 150                 155                 160

Leu Ala Phe Ile Gly Ala Phe Ile Trp Ala Ala Tyr Cys Thr Val Thr
                165                 170                 175

Asn Lys Tyr Ala Arg Gly Phe Asn Gly Ile Thr Val Phe Val Leu Leu
            180                 185                 190

Thr Gly Ala Ser Leu Trp Val Tyr Tyr Phe Leu Thr Pro Gln Pro Glu
        195                 200                 205

Met Ile Phe Ser Thr Pro Val Met Ile Lys Leu Ile Ser Ala Ala Phe
    210                 215                 220

Thr Leu Gly Phe Ala Tyr Ala Ala Trp Asn Val Gly Ile Leu His Gly
225                 230                 235                 240

Asn Val Thr Ile Met Ala Val Gly Ser Tyr Phe Thr Pro Val Leu Ser
                245                 250                 255

Ser Ala Leu Ala Ala Val Leu Leu Ser Ala Pro Leu Ser Phe Ser Phe
            260                 265                 270

Trp Gln Gly Ala Leu Met Val Cys Gly Gly Ser Leu Leu Cys Trp Leu
        275                 280                 285

Ala Thr Arg Arg Gly

    290

<210>  15
<211>  1650
<212>  DNA
<213>  Escherichia coli

<220>
<221>  CDS
<222>  (1)..(1650)
<223>  yjcG-Kodierregion

<400>  15
atg aaa aga gtt ctg acg gcg ctt gcc gcc aca ctc cct ttc gca gct        48
Met Lys Arg Val Leu Thr Ala Leu Ala Ala Thr Leu Pro Phe Ala Ala
1               5                   10                  15

aac gcc gcg gat gct att agc ggg gcc gta gag cgc cag cca acg aac        96
Asn Ala Ala Asp Ala Ile Ser Gly Ala Val Glu Arg Gln Pro Thr Asn
            20                  25                  30

tgg cag gcg att att atg ttc ctg att ttc gtc gtg ttt acg ctc ggc       144
Trp Gln Ala Ile Ile Met Phe Leu Ile Phe Val Val Phe Thr Leu Gly
        35                  40                  45

att acc tac tgg gca tca aaa cgc gta cgt tct cgt agc gac tac tac       192
Ile Thr Tyr Trp Ala Ser Lys Arg Val Arg Ser Arg Ser Asp Tyr Tyr
    50                  55                  60

acc gca ggc ggc aat atc act ggc ttc cag aac ggg ctg gcg att gcc       240
Thr Ala Gly Gly Asn Ile Thr Gly Phe Gln Asn Gly Leu Ala Ile Ala
65                  70                  75                  80

ggg gac tat atg tcc gcc gcc tca ttc ttg ggg atc tcc gcg ctg gtg       288
Gly Asp Tyr Met Ser Ala Ala Ser Phe Leu Gly Ile Ser Ala Leu Val
                85                  90                  95

ttt acc tcc ggc tat gac ggc tta att tac tcg ctg ggc ttc ctg gtg       336
Phe Thr Ser Gly Tyr Asp Gly Leu Ile Tyr Ser Leu Gly Phe Leu Val
            100                 105                 110

ggc tgg ccg atc att ttg ttc ctg att gcc gaa cgt ctg cgt aac ctg       384
Gly Trp Pro Ile Ile Leu Phe Leu Ile Ala Glu Arg Leu Arg Asn Leu
        115                 120                 125

ggg cgc tac acc ttt gcc gat gtg gcc tct tac cgt ctg aaa caa ggg       432
Gly Arg Tyr Thr Phe Ala Asp Val Ala Ser Tyr Arg Leu Lys Gln Gly
    130                 135                 140

ccg att cgt att ctt tcg gcc tgt ggt tct ctg gtg gtg gtg gcg ctt       480
Pro Ile Arg Ile Leu Ser Ala Cys Gly Ser Leu Val Val Val Ala Leu
145                 150                 155                 160

tac ctt atc gcc cag atg gtg ggc gca ggt aaa ctg atc gag ctg ctg       528
Tyr Leu Ile Ala Gln Met Val Gly Ala Gly Lys Leu Ile Glu Leu Leu
                165                 170                 175

ttt ggc ctt aac tat cac att gcg gtg gtg ctg gtc ggc gtg ctg atg       576
Phe Gly Leu Asn Tyr His Ile Ala Val Val Leu Val Gly Val Leu Met
            180                 185                 190

atg atg tac gtc ctg ttc ggc ggc atg ctg gcg acc acc tgg gtg caa       624
Met Met Tyr Val Leu Phe Gly Gly Met Leu Ala Thr Thr Trp Val Gln
        195                 200                 205

att atc aaa gcc gtg ctg ttg ctg ttc ggt gcc agc ttt atg gcc ttt        672
Ile Ile Lys Ala Val Leu Leu Leu Phe Gly Ala Ser Phe Met Ala Phe
    210                 215                 220

atg gtg atg aaa cac gtc ggc ttt agc ttc aac aat ctg ttc agt gaa        720
Met Val Met Lys His Val Gly Phe Ser Phe Asn Asn Leu Phe Ser Glu
225                 230                 235                 240

gcg atg gcg gta cac ccg aaa ggt gtc gac atc atg aag ccg ggc ggg        768
Ala Met Ala Val His Pro Lys Gly Val Asp Ile Met Lys Pro Gly Gly
                245                 250                 255

ctg gtg aaa gat ccg atc tcc gcg ctc tct ctg ggt ctg gga ctg atg        816
Leu Val Lys Asp Pro Ile Ser Ala Leu Ser Leu Gly Leu Gly Leu Met
            260                 265                 270

ttt ggt acg gcg ggc ttg ccg cac att ctg atg cgc ttc ttt aca gtc        864
Phe Gly Thr Ala Gly Leu Pro His Ile Leu Met Arg Phe Phe Thr Val
        275                 280                 285

agc gat gcc cgc gaa gca cgt aag agc gtg ttc tac gcc acc ggg ttt        912
Ser Asp Ala Arg Glu Ala Arg Lys Ser Val Phe Tyr Ala Thr Gly Phe
    290                 295                 300

atg ggc tac ttc tat att ctg acc ttt att atc ggc ttc ggc gcg atc        960
Met Gly Tyr Phe Tyr Ile Leu Thr Phe Ile Ile Gly Phe Gly Ala Ile
305                 310                 315                 320

atg ctg gtt ggt gcg aat ccg gaa tat aaa gac gcg gcg ggc cat ctg       1008
Met Leu Val Gly Ala Asn Pro Glu Tyr Lys Asp Ala Ala Gly His Leu
                325                 330                 335

att ggt ggt aac aac atg gcg gcc gtt cac ctg gcg aat gca gtg ggc       1056
Ile Gly Gly Asn Asn Met Ala Ala Val His Leu Ala Asn Ala Val Gly
            340                 345                 350

ggc aac ctg ttc ctc ggt ttt att tca gcg gtt gct ttc gcc act atc       1104
Gly Asn Leu Phe Leu Gly Phe Ile Ser Ala Val Ala Phe Ala Thr Ile
        355                 360                 365

ctc gcg gtg gtt gcg ggt ctg acg ctg gcg ggc gca tcc gcg gtt tcg       1152
Leu Ala Val Val Ala Gly Leu Thr Leu Ala Gly Ala Ser Ala Val Ser
    370                 375                 380

cat gac ttg tac gct aac gtc ttc aaa aaa ggc gcg acc gaa cgt gaa       1200
His Asp Leu Tyr Ala Asn Val Phe Lys Lys Gly Ala Thr Glu Arg Glu
385                 390                 395                 400

gag ctg cgg gta tca aaa atc acc gta ctg atc ctc ggc gtg att gcg       1248
Glu Leu Arg Val Ser Lys Ile Thr Val Leu Ile Leu Gly Val Ile Ala
                405                 410                 415

att atc ctc ggc gtg ctg ttt gag aat cag aac atc gcc ttt atg gtg       1296
Ile Ile Leu Gly Val Leu Phe Glu Asn Gln Asn Ile Ala Phe Met Val
            420                 425                 430

ggg ctg gcg ttt gcc atc gcg gcg agc tgt aac ttc ccg atc att ctg       1344
Gly Leu Ala Phe Ala Ile Ala Ala Ser Cys Asn Phe Pro Ile Ile Leu
        435                 440                 445

ctt tct atg tac tgg tcg aaa ctg acc acg cgt ggc gcg atg atg ggt       1392
Leu Ser Met Tyr Trp Ser Lys Leu Thr Thr Arg Gly Ala Met Met Gly
    450                 455                 460

ggc tgg ctg ggg ctg att acc gca gta gta ctg atg atc ctc ggc ccg       1440

Gly Trp Leu Gly Leu Ile Thr Ala Val Val Leu Met Ile Leu Gly Pro
465                 470                 475                 480

acg att tgg gta cag atc ctt ggt cac gaa aaa gcc atc ttc ccg tat      1488
Thr Ile Trp Val Gln Ile Leu Gly His Glu Lys Ala Ile Phe Pro Tyr
                485                 490                 495

gaa tac ccg gcg ctg ttc tct atc acc gtg gca ttc ctc ggc atc tgg      1536
Glu Tyr Pro Ala Leu Phe Ser Ile Thr Val Ala Phe Leu Gly Ile Trp
            500                 505                 510

ttc ttc tcg gca acc gat aac tca gcg gaa ggc gcg cgt gag cgt gaa      1584
Phe Phe Ser Ala Thr Asp Asn Ser Ala Glu Gly Ala Arg Glu Arg Glu
        515                 520                 525

ctg ttc cgc gcg cag ttt atc cgc tcc cag acc ggc ttt ggc gtt gag      1632
Leu Phe Arg Ala Gln Phe Ile Arg Ser Gln Thr Gly Phe Gly Val Glu
    530                 535                 540

caa ggc cgc gcg cat taa                                              1650
Gln Gly Arg Ala His
545

<210>  16
<211>  549
<212>  PRT
<213>  Escherichia coli

<400>  16

Met Lys Arg Val Leu Thr Ala Leu Ala Ala Thr Leu Pro Phe Ala Ala
1               5                   10                  15

Asn Ala Ala Asp Ala Ile Ser Gly Ala Val Glu Arg Gln Pro Thr Asn
            20                  25                  30

Trp Gln Ala Ile Ile Met Phe Leu Ile Phe Val Val Phe Thr Leu Gly
        35                  40                  45

Ile Thr Tyr Trp Ala Ser Lys Arg Val Arg Ser Arg Ser Asp Tyr Tyr
    50                  55                  60

Thr Ala Gly Gly Asn Ile Thr Gly Phe Gln Asn Gly Leu Ala Ile Ala
65                  70                  75                  80

Gly Asp Tyr Met Ser Ala Ala Ser Phe Leu Gly Ile Ser Ala Leu Val
                85                  90                  95

Phe Thr Ser Gly Tyr Asp Gly Leu Ile Tyr Ser Leu Gly Phe Leu Val
            100                 105                 110

Gly Trp Pro Ile Ile Leu Phe Leu Ile Ala Glu Arg Leu Arg Asn Leu
        115                 120                 125

Gly Arg Tyr Thr Phe Ala Asp Val Ala Ser Tyr Arg Leu Lys Gln Gly
    130                 135                 140

Pro Ile Arg Ile Leu Ser Ala Cys Gly Ser Leu Val Val Val Ala Leu
145                 150                 155                 160

Tyr Leu Ile Ala Gln Met Val Gly Ala Gly Lys Leu Ile Glu Leu Leu
                165                 170                 175

Phe Gly Leu Asn Tyr His Ile Ala Val Val Leu Val Gly Val Leu Met
            180                 185                 190

Met Met Tyr Val Leu Phe Gly Gly Met Leu Ala Thr Thr Trp Val Gln
        195                 200                 205

Ile Ile Lys Ala Val Leu Leu Leu Phe Gly Ala Ser Phe Met Ala Phe
    210                 215                 220

Met Val Met Lys His Val Gly Phe Ser Phe Asn Asn Leu Phe Ser Glu
225                 230                 235                 240

Ala Met Ala Val His Pro Lys Gly Val Asp Ile Met Lys Pro Gly Gly
                245                 250                 255

Leu Val Lys Asp Pro Ile Ser Ala Leu Ser Leu Gly Leu Gly Leu Met
            260                 265                 270

Phe Gly Thr Ala Gly Leu Pro His Ile Leu Met Arg Phe Phe Thr Val
        275                 280                 285

Ser Asp Ala Arg Glu Ala Arg Lys Ser Val Phe Tyr Ala Thr Gly Phe
    290                 295                 300

Met Gly Tyr Phe Tyr Ile Leu Thr Phe Ile Ile Gly Phe Gly Ala Ile
305                 310                 315                 320

Met Leu Val Gly Ala Asn Pro Glu Tyr Lys Asp Ala Ala Gly His Leu
                325                 330                 335

Ile Gly Gly Asn Asn Met Ala Ala Val His Leu Ala Asn Ala Val Gly
            340                 345                 350

Gly Asn Leu Phe Leu Gly Phe Ile Ser Ala Val Ala Phe Ala Thr Ile
        355                 360                 365

Leu Ala Val Val Ala Gly Leu Thr Leu Ala Gly Ala Ser Ala Val Ser
    370                 375                 380

His Asp Leu Tyr Ala Asn Val Phe Lys Lys Gly Ala Thr Glu Arg Glu
385                 390                 395                 400

Glu Leu Arg Val Ser Lys Ile Thr Val Leu Ile Leu Gly Val Ile Ala

405                 410                 415

Ile Ile Leu Gly Val Leu Phe Glu Asn Gln Asn Ile Ala Phe Met Val
            420                 425                 430

Gly Leu Ala Phe Ala Ile Ala Ala Ser Cys Asn Phe Pro Ile Ile Leu
        435                 440                 445

Leu Ser Met Tyr Trp Ser Lys Leu Thr Thr Arg Gly Ala Met Met Gly
    450                 455                 460

Gly Trp Leu Gly Leu Ile Thr Ala Val Val Leu Met Ile Leu Gly Pro
465                 470                 475                 480

Thr Ile Trp Val Gln Ile Leu Gly His Glu Lys Ala Ile Phe Pro Tyr
                485                 490                 495

Glu Tyr Pro Ala Leu Phe Ser Ile Thr Val Ala Phe Leu Gly Ile Trp
            500                 505                 510

Phe Phe Ser Ala Thr Asp Asn Ser Ala Glu Gly Ala Arg Glu Arg Glu
        515                 520                 525

Leu Phe Arg Ala Gln Phe Ile Arg Ser Gln Thr Gly Phe Gly Val Glu
    530                 535                 540

Gln Gly Arg Ala His
545

## Beschreibung

Diese Erfindung betrifft ein Verfahren zur fermentativen Herstellung von L-Aminosäuren, insbesondere L-Threonin, L-Tryptophan, L-Lysin, L-Valin und L-Homoserin, unter Verwendung von rekombinanten Mikroorganismen der Familie Enterobacteriaceae, in denen das malI-Gen, welches für den Transkriptionsregulator MalI kodiert, verstärkt, insbesondere überexprimiert wird, und diese Mikroorganismen.

### Stand der Technik

L-Aminosäuren, insbesondere L-Threonin, L-Tryptophan, L-Lysin, L-Valin und L-Homoserin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2^{nd} edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)) .

Der Transkriptionsregulator MalI ist ein Repressor vom LacI-Typ, eine Transposoninsertion im malI-Gen führt zur Expression des divergent zu malI transkribierten malXY-Operons, wobei MalY für die Repression von MalT-abhängigen Genen verantwortlich ist (Reidl et al., Journal of Bacteriology, 173(15), 4862-76 (1991)). MalT, ein transkriptionaler Aktivator, kontrolliert die 10 Gene des Maltose-Systems von Escherichia coli, die die Aufnahme und den Stoffwechsel von Maltose und Maltodextrin regeln (Boos, W. and Shuman, H.A.; Microbiology and Molecular Biology Reviews 16: 204-229 (1998)).

Die Nukleotidsequenz der Wildform des für den Transkriptionsregulator MalI von Escherichia coli kodierenden Gens wurde von Reidl et al. (Journal of Bacteriology, 171(9), 4888-99 (1989)) bestimmt und ist in der Datenbank des National Center for Biotechnology Information (NCBI) der National Library of Medicin (Bethesda, MD, USA) unter den Zugangsnummern (accession number) M28539 und U00096 (Region: complement(1696176-1697204)) allgemein verfügbar.

### Aufgabe der Erfindung

Die Erfinder haben sich die Aufgabe gestellt, neue Maßnahmen zur Herstellung erhöhter Mengen von L-Aminosäuren, insbesondere L-Threonin, L-Tryptophan, L-Lysin, L-Valin und L-Homoserin, bereitzustellen.

### Beschreibung der Erfindung

Gegenstand der Erfindung sind rekombinante L-Aminosäure produzierende Mikroorganismen der Familie Enterobacteriaceae, die ein verstärktes oder überexprimiertes malI-Gen, das für den Transkriptionsregulator MalI kodiert, oder für dessen Genprodukt kodierende Nukleotidsequenzen enthalten, und die vermehrt L-Aminosäuren, insbesondere L-Threonin, L-Tryptophan, L-Lysin, L-Valin und L-Homoserin, produzieren und in der Zelle oder im Medium anreichern.

Als Ausgangspunkt für den Vergleich dienen jeweils die für das malI-Gen nicht rekombinanten Mikroorganismen, die kein verstärktes malI-Gen enthalten, und an denen die erfindungsgemäße Verstärkung oder Überexpression nicht durchgeführt wurde.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 70% oder zu mindestens 80% oder mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2 und SEQ ID No. 4, bevorzugt SEQ ID No. 2, wobei das Polypeptid die Aktivität des Transkriptionsregulators MalI besitzt.

Die genannten Mikroorganismen enthalten isolierte Polynukleotide, ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1 oder SEQ ID No. 3, die funktionsneutrale Sinnmutanten enthält, wobei die Polynukleotide für einen Transkriptionsregulator kodieren.

Gegenstand der Erfindung ist ebenso ein Verfahren zur fermentativen Herstellung von L-Aminosäuren, insbesondere L-Threonin, L-Tryptophan, L-Lysin, L-Valin und L-Homoserin, unter Verwendung von rekombinanten Mikroorganismen der Familie Enterobacteriaceae, nicht von Wildtypstämmen, die insbesondere bereits vor der erfindungsgemäßen Verstärkung oder Überexpression L-Aminosäuren produzieren, und in denen mindestens das malI-Gen oder für dessen Genprodukt kodierende Nukleotidsequenzen verstärkt wird bzw. werden.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Threonin, L-Tryptophan, L-Lysin, L-Valin und L-Homoserin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Ein Gen oder Allel ist chemisch ein Polynukleotid. Ein anderer Begriff hierfür ist Nukleinsäure, insbesondere Desoxyribonukleinsäure.

Die Begriff Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Gegenstand der Erfindung ist ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man das malI-Gen oder für dessen Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten malI-Gen, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der L-Aminosäure-ausscheidenden Stämme der Familie Enterbacteriaceae, welche ein verstärktes malI-Gen enthalten, werden bevorzugt Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür kann auch der Ausdruck "produzieren" verwendet werden. Insbesondere besitzen die für die Maßnahmen der Erfindung eingesetzten Stämme die Fähigkeit > (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-ausscheidenden Stämme produzieren eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Tryptophan, L-Lysin, L-Valin und L-Homoserin. Der Begriff L-Aminosäure oder Aminosäure umfasst auch deren Salze.

Der Begriff "eine oder im Wesentlichen eine Aminosäure" berücksichtigt, dass zusätzlich zu der gewünschten L-Aminosäure eine oder mehrer weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Als Elternstamm geeigneter, L-Homoserin ausscheidender bzw. produzierender Stamm der Gattung Escherichia, insbesondere der Art Escherichia coli ist beispielsweise zu nennen:
Escherichia coli NZ10rhtA23/pAL4 (US 6,960,455).

Als Elternstamm geeigneter, L-Lysin ausscheidender bzw. produzierender Stamm der Gattung Escherichia, insbesondere der Art Escherichia coli ist beispielsweise zu nennen:
Escherichia coli pDA1/TOC21R (= CNCM 1-167) (FR-A-2511032).

Bei den der Erfindung zugrunde liegenden Arbeiten wurde gefunden, dass Mikroorganismen der Familie Enterobacteriaceae nach Überexpression des Gens malI, oder dessen Allelen, vermehrt L-Aminosäuren, insbesondere L-Threonin, L-Tryptophan, L-Lysin, L-Valin und L-Homoserin, produzieren und in der Zelle oder im Medium anreichern

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus dem ebenfalls zur Familie Enterobacteriaceae gehörenden Shigella flexneri ist die Nukleotidsequenz für das malI-Gen ebenso bekannt (Accession No.: NC_004337 (REGION: complement(1674868-1675896)).

Das malI-Gen von Escherichia coli K12 wird unter anderem durch folgende Angaben beschrieben:

Das Genprodukt des malI-Gens ist annotiert als Transkriptionsregulator, ein Repressor aus der LacI/GalR-Familie. Das Gen liegt 174 Nukleotide stromaufwärts des divergent orientierten malXY-Operons. Der Repressor reguliert den Transport von Maltose/Glukose, ist negativ autorreguliert und reprimiert die Expression des malXY-Operons, das für eine Maltose-Glukose PTS Permease (MalX) und ein bifunctionales Protein (MalY) kodiert, das mit dem Transkriptionsaktivator MalT interagiert (Reidl et al., Journal of Bacteriology, 171(9), 4888-99 (1989); Reidl et al., Journal of Bacteriology, 173(15), 4862-76 (1991)).
Accession No.: U00096 (Region: complement(1696176-1697204))
Alternativer Genname: b1620

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum malI-Gen von Escherichia coli unter der SEQ ID No. 1 und die bekannten Sequenz zum malI-Gen von Shigella flexneri unter der SEQ ID No. 3 dargestellt. Die von diesen Leserahmen kodierten Proteine sind als SEQ ID No. 2 und SEQ ID No. 4 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des malI-Gens, welche funktionsneutrale Sinnmutationen enthalten, zählen unter anderem solche, die zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1 oder SEQ ID No. 3 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1 oder SEQ ID No. 3 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558) .

Zur Erzielung einer Verstärkung können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden.

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das malI-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus) .

Zur Erzeugung von malI-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im malI-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene malI-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten malI-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten malI-Allele durch Konjugation oder Transduktion in verschiedene Stämme zu überführen.

Die Mutationen von malI können ebenfalls in Expressionsplasmiden durchgeführt werden, so dass die resultierenden Proteine überproduziert werden können.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die erhöhte Produktion von L-Aminosäuren, insbesondere L-Threonin, L-Tryptophan, L-Lysin, L-Valin und L-Homoserin mit Stämmen der Familie Enterobacteriaceae vorteilhaft sein, zusätzlich zur Verstärkung des malI-Gens, ein oder mehrere Enzym(e) des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So kann es für die Produktion von L-Trytophan mit Stämmen der Familie Enterobacteriaceae vorteilhaft sein, zusätzlich zur Verstärkung des malI-Gens, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

So können beispielsweise für die Produktion von L-Threonin gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986); WO 95/11985),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA), der besseren Übersichtlichkeit halber ist die Sequenz des yjcG-ORFs unter der SEQ ID No. 15 und die des von diesem Leserahmen kodierten Proteins als SEQ ID No. 16 dargestellt),
- das Genprodukt des offenen Leserahmens (ORF) yibD von Escherichia coli (Accession Number AE000439 des National Center for Biotechnology Information (NCBI, Bethesda, MD, USA)), der besseren Übersichtlichkeit halber ist die Sequenz des yibD-ORFs unter der SEQ ID No. 9 und die des von diesem Leserahmen kodierten Proteins als SEQ ID No. 10 dargestellt),
- das Genprodukt des offenen Leserahmens (ORF) yaaU von Escherichia coli (Accession Number NC000913 (Region 45807-47138) des National Center for Biotechnology Information (NCBI, Bethesda, MD, USA), der besseren Übersichtlichkeit halber ist die Sequenz des yaaU-ORFs unter der SEQ ID No. 11 und die des von diesem Leserahmen kodierten Proteins als SEQ ID No. 12 dargestellt)
verstärkt, insbesondere überexprimiert werden.

Desweiteren können beispielsweise für die Produktion von L-Tryptophan, gleichzeitig eines oder mehrere der Gene, ausgewählt aus der Gruppe
- mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5, 168, 056),
- das für die Transketolase B kodierende tktB-Gen (US5, 168, 056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA), der besseren Übersichtlichkeit halber ist die Sequenz des yddG-ORFs unter der SEQ ID No. 13 und die des von diesem Leserahmen kodierten Proteins als SEQ ID No. 14 dargestellt)
verstärkt, insbesondere überexprimiert werden.

Weiterhin kann es für die Produktion von L-Aminosäuren, insbesondere L-Threonin, L-Tryptophan, L-Lysin, L-Valin und L-Homoserin vorteilhaft sein, zusätzlich zur Verstärkung des malI-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist und
- das für die Aspartat Ammonium-Lyase kodierende aspA-Gen (WO 03/008603)
abgeschwächt, insbesondere ausgeschaltet oder die Expression verringert werden. Diese Massnahmen werden gegebenenfalls zusätzlich zu bzw. in geeigneter Kombination mit den angegebenen Massnahmen zur Erhöhung der Threonin-Produktion durchgeführt.

Weiterhin kann es für die Produktion von L-Tryptophan vorteilhaft sein, zusätzlich zur Verstärkung des malI-Gens, eines oder mehrere der Gene ausgewählt aus der Gruppe
- das für die Tryptophanase kodierende tnaA-Gen (US4, 371, 614),
- das für den Repressor des trp-Operons kodierende trpR-Gen (US4,371,614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),
- das für das Tryptophan spezifische Transportprotein kodierende mtr-Gen (US,756, 345),
- das für die Tryptophan-Permease kodierende tnaB-Gen (US5, 756, 345),
- das für den Transporter für aromatische Aminosäuren kodierende aroP-Gen (US5,756,345),
- das für die L-Serin Deaminase kodierende sdaA-Gen (EP0149539),
- das für die Glukose-6-Phosphat-Isomerase kodierende pgi-Gen (WO87/01130),
- das für die Tyrosin-Aminotransferase kodierende tyrB-Gen (WO87/01130)
abzuschwächen, insbesondere auszuschalten oder die Expression zu verringern. Diese Massnahmen werden gegebenenfalls zusätzlich zu bzw. in geeigneter Kombination mit den angegebenen Massnahmen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Inhaltsstoffe des/ der eingesetzten Fermentationsmediums/Fermentationsmedien bzw. Anteile der Einsatzstoffe wie beispielsweise Vitamine wie Thiamin oder Salze wie Magnesiumsulfat. Unter den Punkten a, c und d sind die "weiteren" Bestandteile der Fermentationsbrühe benannt.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥ 60%, ≥ 70%, ≥ 80%, ≥ 90% oder ≥ 95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin, L-Tryptophan, L-Lysin, L-Valin und L-Homoserin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pTrc99AmalI

Das Gen malI aus E. coli K12 wurde unter Anwendung der Polymerase-Kettenreaktion (PCR) sowie synthetischer Oligonukleotiden amplifiziert. Ausgehend von der Nukleotidsequenz des malI-Gens in E. coli K12 MG1655 (Accession Number U00096 (Region: complement(1696176-1697204)) wurden PCR-Primer synthetisiert (MWG Biotech, Ebersberg, Deutschland). Die Sequenzen der Primer wurden so modifiziert, dass Erkennungsstellen für Restriktionsenzyme entstanden. Für den malI1-Primer wurde die Erkennungssequenz für XbaI und für den malI2-Primer die Erkennungssequenz für HindIII gewählt, die in der unten dargestellten Nukleotidabfolge durch Unterstreichen markiert sind:
malI1: 5' - CGT TTT ATC TAG AAC ATA ATC AGG GAG TAG G - 3' (SEQ ID No. 5)
malI2: 5' - CAC TAC CAG GTA TAG AAG CTT AAG AGA GG - 3' (SEQ ID No. 6)

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein ca. 1100 bp großes DNA-Fragment konnte mit den spezifischen Primern unter Standard-PCR-Bedingungen (Innis et al. (1990) PCR Protocols. A Guide to Methods and Applications, Academic Press) mit der Vent-DNA-Polymerase (New England Biolabs, Frankfurt, Deutschland) amplifiziert werden.

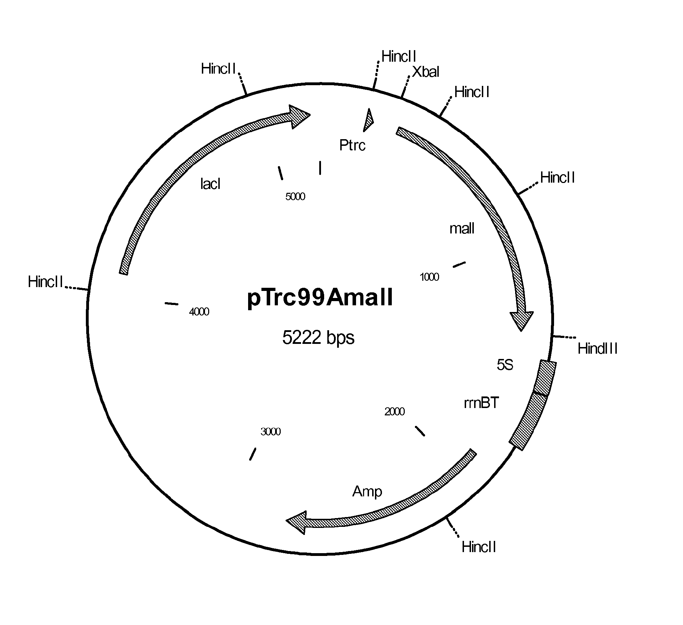

Das PCR-Produkt (die Sequenz ist dargestellt in SEQ ID No. 7, die Aminosäuresequenz des korrespondierenden Proteins in SEQ ID No. 8 entspricht der in SEQ ID No. 2) wurde mit den Restriktionsenzymen XbaI und HindIII gespalten und mit dem Vektor pTrc99A (Pharmacia Biotech, Uppsala, Schweden), der mit den Enzymen XbaI und HindIII verdaut worden ist, ligiert. Der E. coli Stamm TOP10 (Invitrogen, Paisley, UK) wurde mit dem Ligationsansatz transformiert und Plasmid tragende Zellen auf LB Agar, der mit 50 µg/ml Ampicillin versetzt ist, selektioniert. Die erfolgreiche Klonierung konnte nach der Plasmid DNA Isolierung durch die Kontrollspaltung mit den Enzymen HincI, HindIII und XbaI nachgewiesen werden. Das Plasmid wird als pTrc99AmalI (Figur 1) bezeichnet.

### Beispiel 2

### Herstellung von L-Threonin mit dem Stamm MG442/pTrc99AmalI

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wurde mit dem in Beispiel 1 beschriebenen Expressionsplasmid pTrc99AmalI und mit dem Vektor pTrc99A transformiert und auf LB Agar mit 50 µg/ml Ampicillin Plasmid tragende Zellen selektioniert. Auf diese Weise entstehen die Stämme MG442/pTrc99AmalI und MG442/pTrc99A. Ausgewählte Einzelkolonien wurden anschließend auf Minimalmedium mit der folgenden Zusammensetzung: 3,5 g/l Na₂HPO₄*2H₂O, 1,5 g/l KH₂PO₄, 1 g/l NH₄Cl, 0, 1 g/l MgSO₄*7H₂O, 2 g/l Glucose, 20 g/l Agar, 50 mg/l Ampicillin, weiter vermehrt. Die Bildung von L-Threonin wurde in batch Kulturen von 10 ml, die in 100 ml Erlenmeierkolben enthalten sind, überprüft. Dazu wurde 10 ml Vorkulturmedium der folgenden Zusammensetzung: 2 g/l Hefeextrakt, 10 g/l (NH₄)₂SO₄, 1 g/l KH₂PO₄, 0, 5 g/l MgSO₄*7H₂O, 15 g/l CaCO₃, 20 g/l Glucose, 50 mg/l Ampicillin, beimpft und für 16 Stunden bei 37°C und 180 rpm auf einem ESR Inkubator der Firma Kühner AG (Birsfelden, Schweiz) inkubiert.

Je 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin) überimpft und für 48 Stunden bei 37°C inkubiert. Die Bildung von L-Threonin durch den Ausgangsstamm MG442 wird in der gleichen Weise überprüft, wobei jedoch keine Zugabe von Ampicillin zum Medium erfolgt. Nach der Inkubation wurde die optische Dichte (OD) der Kultursuspension mit einem LP2W-Photometer der Firma Dr. Lange (Düsseldorf, Deutschland) bei einer Messwellenlänge von 660 nm bestimmt.

Anschließend wurde die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442 | 5,6 | 1,4 |
| MG442/pTrc99A | 3,0 | 1,3 |
| MG442/pTrc99AmalI | 5,4 | 2,2 |

### Kurze Beschreibung der Figur:

- Figur 1:: Karte des das malI-Gen enthaltenden Plasmids pTrc99AmalI

Längenangaben sind als ca.-Angaben aufzufassen. Die verwendeten Abkürzungen und Bezeichnungen haben folgende Bedeutung:
- Amp: Ampicillinresistenzgen
- lacI: Gen für das Repressorprotein des trc-Promotors
- Ptrc: trc-Promotorregion, IPTG-induzierbar
- malI: Kodierregion des malI-Gens
- 5S: 5S rRNA-Region
- rrnBT: rRNA-Terminator-Region

Die Abkürzungen für die Restriktionsenzyme haben folgende Bedeutung
- HincII: Restriktionsendonuklease aus Haemophilus influenzae Rc
- HindIII: Restriktionsendonuklease aus Haemophilus influenzae Rd
- XbaI: Restriktionsendonuklease aus Xanthomonas campestris

## Patentansprüche

1. Rekombinante L-Aminosäure produzierende Mikroorganismen der Familie Enterobacteriaceae, die ein verstärktes oder überexprimiertes malI-Gen enthalten, welches für den Transkriptionsregulator MalI kodiert.

2. Mikroorganismen gemäss Anspruch 1, in denen ein dem malI-Gen entsprechendes Polynukleotid verstärkt wird, das für ein Polypeptid kodiert, dessen Aminosäuresequenz zu mindestens 90% identisch ist mit einer Aminosäuresequenz, ausgewählt aus der Gruppe SEQ ID No. 2 und SEQ ID No. 4, wobei das Polypeptid die Aktivität des Transkriptionsregulators MalI besitzt.

3. Mikroorganismen gemäss Anspruch 2, **dadurch gekennzeichnet, dass** sie ein dem malI-Gen entsprechendes isoliertes Polynukleotid enthalten, ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1 oder SEQ ID No. 3, die funktionsneutrale Sinnmutanten enthält,
wobei die Polynukleotide für einen Transkriptionsregulator kodieren.

4. Mikroorganismen gemäss Anspruch 2, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2 und SEQ ID No. 4.

5. Mikroorganismen gemäss Anspruch 2, **dadurch gekennzeichnet, dass** das Polypeptid die Aminosäuresequenz aufweist, die 100% identisch ist mit einer der Sequenzen, ausgewählt aus der Gruppe SEQ ID No. 2 oder SEQ ID No. 4.

6. Mikroorganismus gemäss den Ansprüchen 1 bis 5, **dadurch gekennzeichnet, dass** sie durch Transformation, Transduktion, Konjugation oder eine Kombination dieser Methoden hergestellt werden, mit einem Vektor, der das malI-Gen, ein Allel dieses Gens oder Teile davon und/oder einen Promotor enthält.

7. Mikroorganismen gemäss den Ansprüchen 1 bis 6, in denen die Kopienzahl des malI-Gens oder der Allele um mindestens 1 erhöht vorliegt.

8. Mikroorganismen gemäss Anspruch 7, **dadurch gekennzeichnet, dass** die Erhöhung der Kopienzahl des malI-Gens um mindestens 1 durch Integration des Gens oder der Allele in das Chromosom der Mikroorganismen bewirkt wird.

9. Mikroorganismen gemäss Anspruch 7, **dadurch gekennzeichnet, dass** die Erhöhung der Kopienzahl des malI-Gens um mindestens 1 durch einen extrachromosomal replizierenden Vektor erzielt wird.

10. Mikroorganismen gemäss den Ansprüchen 1 bis 9, **dadurch gekennzeichnet, dass** man zur Erzielung der Verstärkung
a) die Promotor- und Regulationsregion oder die Ribosomenbindungsstelle stromaufwärts des malI-Gens mutiert, oder
b) Expresssionskassetten oder Promotoren stromaufwärts des malI-Gens einbaut.

11. Mikroorganismen gemäss den Ansprüchen 1 bis 9, **dadurch gekennzeichnet, dass** die Expression des malI-Gens unter der Kontrolle eines die Expression des Gens verstärkenden Promotors steht.

12. Mikroorganismen gemäss den Ansprüchen 1 bis 11, **dadurch gekennzeichnet, dass** durch die Verstärkung des malI-Gens die Konzentration oder Aktivität des malI-Genproduktes (Proteins) um mindestens 10% erhöht sind, bezogen auf die Aktivität oder Konzentration des Genproduktes im für das malI-Gen nicht rekombinanten Mikroorganismus oder Elternstamm.

13. Mikroorganismen gemäss den Ansprüchen 1 bis 12, **dadurch gekennzeichnet, dass** die Mikroorganismen aus den Gattungen Escherichia, Erwinia, Providencia und Serratia ausgewählt sind.

14. Mikroorganismen gemäss Anspruch 13, **dadurch gekennzeichnet, dass** zusätzlich weitere Gene des Biosyntheseweges der gewünschten L-Aminosäure verstärkt, insbesondere überexprimiert vorliegen.

15. Mikroorganismen gemäss den Ansprüchen 1 bis 14, **dadurch gekennzeichnet, dass** sie L-Threonin, L-Tryptophan, L-Lysin, L-Valin oder L-Homoserin produzieren.

16. Verfahren zur Herstellung von L-Aminosäuren durch Fermentation von rekombinanten Mikroorganismen der Familie Enterobacteriaceae, **dadurch gekennzeichnet, dass** man
a) die die gewünschte L-Aminosäure produzierenden Mikroorganismen gemäß den Ansprüchen 1 bis 15 in einem Medium kultiviert unter Bedingungen, bei denen die gewünschte L-Aminosäure im Medium oder in den Zellen angereichert wird, und
b) die gewünschte L-Aminosäure isoliert, wobei die weiteren Bestandteile der Fermentationsbrühe und/oder die Biomasse in ihrer Gesamtheit oder Anteilen (≥ 0 bis 100%) im isolierten Produkt verbleiben oder vollständig entfernt werden.

17. Verfahren gemäss Anspruch 16 , **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
verstärkt, insbesondere überexprimiert.

18. Verfahren gemäss Anspruch 16, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
18.1 mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
18.2 das für die 3-Phosphoglycerat-Dehydrogenase kodierende serA-Gen,
18.3 das für die Phosphoserinphosphatase kodierende serB-Gen,
18.4 das für die Phosphoserinaminotransferase kodierende serC-Gen,
18.5 das für die L-Tyrosin sensitive DHAP-Synthase kodierende aroF-Gen,
18.6 das für die L-Phenylalanin sensitive DHAP-Synthase kodierende aroG-Gen,
18.7 das für die L-Tryptophan sensitive DHAP-Synthase kodierende aroH-Gen,
18.8 das für die Phosphoenolpyruvat-Synthase kodierende pps-Gen,
18.9 das für die Phosphoenolpyruvat-Carboxykinase kodierende pckA-Gen,
18.10 das für die Transketolase A kodierende tktA-Gen,
18.11 das für die Transketolase B kodierende tktB-Gen, und
18.12 das Genprodukt des offenen Leserahmens (ORF) yddG von Escherichia coli
verstärkt, insbesondere überexprimiert.

19. Verfahren gemäss den Ansprüchen 16 bis 18, **dadurch gekennzeichnet, dass** man Mikroorganismen einsetzt, in denen die Stoffwechselwege zumindest teilweise abgeschwächt sind, die die Bildung der gewünschten L-Aminosäure verringern.

20. Verfahren gemäss den Ansprüchen 16 bis 17 oder 19, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen, und
h) das für die Aspartat Ammonium-Lyase kodierende aspA-Gen
abschwächt, insbesondere ausschaltet oder die Expression verringert.

21. Verfahren gemäss den Ansprüchen 16 oder 18 bis 19, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
21.1 das für die Tryptophanase kodierende tnaA-Gen,
21.2 das für den Repressor des trp-Operons kodierende trpR-Gen,
21.3 das für die Chorismat-Mutase T und Prephenat-Dehydrogenase kodierende tyrA-Gen,
21.4 das für die Chorismat-Mutase P und Prephenat-Dehydrogenase kodierende pheA-Gen,
21.5 das für das Tryptophan spezifische Transportprotein kodierende mtr-Gen,
21.6 das für die Tryptophan-Permease kodierende tnaB-Gen,
21.7 das für den Transporter für aromatische Aminosäuren kodierende aroP-Gen,
21.8 das für die L-Serin Deaminase kodierende sdaA-Gen,
21.9 das für die Glukose-6-Phosphat-Isomerase kodierende pgi-Gen, und
21.10 das für die Tyrosin-Aminotransferase kodierende tyrB-Gen
abschwächt, insbesondere ausschaltet oder die Expression verringert.

22. Verfahren gemäss den Ansprüchen 16 und 19, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin herstellt.

23. Verfahren gemäss den Ansprüchen 16 und 19, **dadurch gekennzeichnet, dass** man L-Aminosäuren, ausgewählt aus der Gruppe L-Threonin, L-Tryptophan, L-Lysin, L-Valin und L-Homoserin herstellt.
